# EUROPEAN PATENT APPLICATION

(11) **EP 2 093 241 A2**
(43) Date of publication of application: **26.08.2009**
(21) Application number: 09250192.3
(22) Date of filing: 26.01.2009
(51) Int. Cl.: C08G 18/32

(54) **Bioabsorbable block copolymer**

(30) Priority: 29.01.2008 US 24296; 20.01.2009 US 356171
(71) Applicant: Confluent Surgical Inc., Waltham, Massachusetts 02451 (US)
(72) Inventor: Bennett, Steven L., Cheshire, CT 06410 (US)
(74) Representative: Soames, Candida Jane

(57) **Abstract**

A bioabsorbable polyurethane block copolymer with having hard and soft segments is formed by the reaction between a diisocyanate such as lysine ethyl ester diisocyanate and a mixture of (i) an aromatic amide or an amino acid derivative and (ii) a polyether glycol and/or an absorbable elastomeric polymer derived from the reaction between dialkylene glycol and a cyclic bioabsorbable monomer such as trimethylene carbonate, caprolactone, or dioxanone.

## Description

### CROSS-REFERENCE TO RELATED APPLICATIONS

This application claims the benefit of and priority to U.S. Provisional Patent Application No. 61/024,296, filed January 29, 2008, the entire disclosure of which is incorporated by reference herein.

### BACKGROUND

### 1. Technical Field

This disclosure relates to a biocompatible and bioabsorbable block copolymer for use in fabricating surgical articles, and more particularly to a block copolymer possessing hard and soft segments.

### 2. Background of Related Art

Bioabsorbable polymers are often used to fabricate surgical articles such as surgical sutures, clips, staples, pins, and other such fastening devices. Other surgical uses for bioabsorbable materials include bone implants, time release drug delivery systems, nerve channels, vascular grafts, wound covering devices and the like. Bioabsorbable materials possess the advantage of being implantable in a body for a temporary period of time without the necessity of a separate surgical operation for their removal. They can be used, for example, to fasten tissue for a period of time sufficient to allow healing, after which the bioabsorbable material naturally degrades and is absorbed.

Natural bioabsorbable materials such as catgut or collagen degrade under the action of enzymolysis. Synthetic polymers such as polygycolide and polylactide degrade under the action of hydrolysis. In either case, however, it is important that the byproducts of the breakdown of the bioabsorbable material be biocompatible. That is, the degradation products of the bioabsorbable material should not cause tissue inflammation or any other adverse body reaction.

U.S. Patent No. 5,120,813 to Ward, Jr. discloses polymeric materials characterized by hard segments and copolymer soft blocks comprising hydrophobic and hydrophilic components. The polymeric materials may be formed into films for use a wound or burn dressings, surgical drapes, semipermeable membranes, and coatings for textiles.

U.S. Patent No. 5,274,074 to Tang et al. discloses polymers containing recurring carbonate linkages for use in fabricating absorbable medical devices.

U.S. Patent No. 5,321,113 to Cooper et al. discloses a copolymer of an aromatic anhydride and an aliphatic ester for use in surgical devices.

### SUMMARY

A bioabsorbable synthetic block copolymer having hard segments and soft segments is disclosed herein. The bioabsorbable block copolymer is made by reacting a biocompatible and biodegradable diisocyanate with the following mixture. The diisocyanate is preferably a derivative of an amino acid such as, for example, lysine ethyl ester diisocyanate. A first component of the mixture which is reacted with the diisocyanate includes an aromatic compound having molecular formula (I): 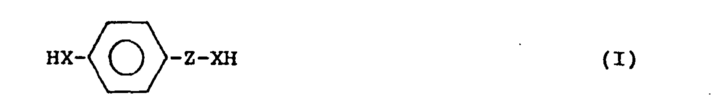 wherein X is oxygen or sulfur, and Z is selected from the group consisting of an alkyl moiety having from 1 to about 6 carbon atoms, an aralkyl moiety having from 7 to about 12 carbon atoms, a moiety having molecular formula (II): 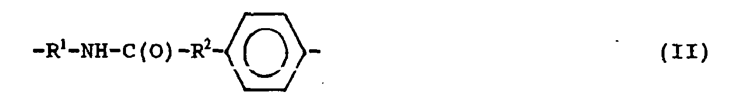 a moiety having molecular formula (III): 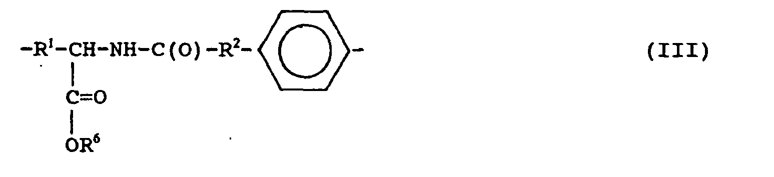 and a moiety having molecular formula (IV):

-R³-CH(NHR⁵) -C(O)O-R⁴- (IV)

wherein R¹, R², R³, and R⁴ are independently selected from the group consisting of an alkyl moiety having from 1 to about 6 carbon atoms and an aralkyl group having from 7 to about 10 carbon atoms, R⁵ is hydrogen or an alkyl group having from 1 to about 6 carbon atoms and R⁶ is an alkyl group of from about 1 to about 18 carbon atoms.

A second component of the mixture includes an aliphatic polyether glycol and/or an absorbable elastomeric polymer derived from the reaction between dialkylene glycol and a cyclic bioabsorbable monomer. The cyclic bioabsorbable monomer includes at least one of: trimethylene carbonate, dioxanone, and caprolactone.

The polyalkylene glycol is selected from polyethylene glycol and polypropylene glycol. The absorbable elastomeric polymer is preferably derived from the reaction between diethylene glycol and trimethylene carbonate.

Alternatively, the bioabsorbable block copolymer can be made by reacting the biodegradable and biocompatible diisocyanate with a mixture containing the second mixture component as described above and with an amino acid derivative having molecular formula (IV):

H-[-X-R⁷-CH(NHR⁸)-C(O)-]ₙ-X-R⁷-CH(NHR⁸)-C(O)-Y-R⁹-OH (V)

wherein X is oxygen or sulfur, R⁷ and R⁹ are independently selected from an alkyl moiety having from 1 to about 6 carbon atoms, aryl, or alkaryl having from 7 to about 12 carbon atoms; R⁸ is hydrogen or an alkyl moiety having from 1 to about 6 carbon atoms, Y is oxygen or -NH-, and n is an integer of from 1 to about 10.

The bioabsorbable block copolymer made in accordance with the materials and method described herein can be used to construct various types of surgical devices such as implants, prosthetics, surgical fasteners, etc. Moreover, various medically useful substances can be coated on or incorporated into the block copolymer to promote healing and prevent infections.

### BRIEF DESCRIPTION OF THE DRAWINGS

Various embodiments are described below with reference to the drawings wherein:
FIG. 1 illustrates a needle-suture combination.
FIG. 2 is a perspective view of a bone pin;
FIG. 3 is a perspective view of a surgical clip; and
FIG. 4 is a perspective view of a surgical staple and retainer.

### DETAILED DESCRIPTION OF PREFERRED EMBODIMENTS

A biocompatible and bioabsorbable synthetic block copolymer having hard segments and soft segments is made reacting a biocompatible and bioabsorbable diisocyanate with a mixture of (i) and (ii) wherein:
(i) is an aromatic compound having the formula 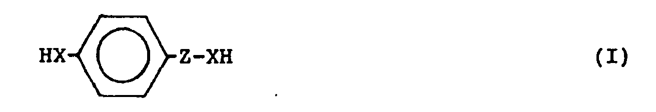 wherein X is oxygen or sulfur, and Z is selected from an alkyl moiety having from 1 to about 6 carbon atoms, an aralkyl moiety having from 7 to about 12 carbon atoms, and is preferably selected from groups having formulas (II), (III), or (IV):

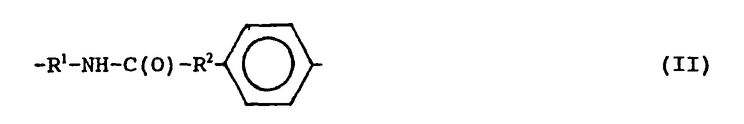

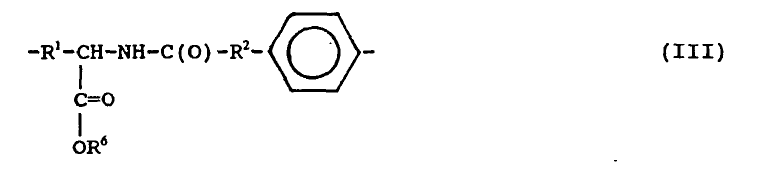

   -R³-CH(NHR⁵)-C(O)O-R⁴- (IV)

   wherein R¹, R², R³, and R⁴ are independently selected from alkyl groups having from 1 to about 6 carbon atoms and aralkyl groups having from 7 to about 10 carbon atoms, R⁵ is hydrogen or an alkyl group having from 1 to about 6 carbon atoms, and R⁶ is an alkyl group of from about 1 to about 18 carbon atoms; and
ii) is an aliphatic polyether glycol and/or an absorbable elastomeric polymer derived from the reaction between dialkylene glycol and one or more cyclic bioabsorbable monomer such as trimethylene carbonate, dioxanone or caprolactone.

A preferred compound having formula I is prepared by reacting a 4-hydroxyphenyl alkanoic acid with a 4-hydroxyphenyl alkylamine. For example, 4-hydroxyphenyl propionic acid, when reacted with 4-hydroxyphenyl ethylamine, produces a compound having the formula 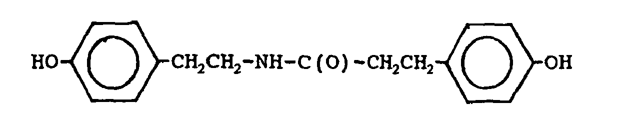 which corresponds to formula I wherein X is oxygen, and Z is a group of formula II wherein R¹ and R² are each -CH₂CH₂-.

Other compounds having formula I can be prepared by reacting tyrosine with a diol compound having the formula HO-R⁴-OH to form a compound having the formula 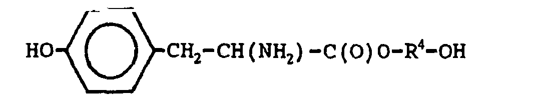 Preferably, a derivative of tyrosine is employed in which R⁵ is an alkyl moiety having from 1 to about 6 carbon atoms.

As an alternative to the compound of formula I, a compound of formula V may be used instead:

H-[-X-R⁷-CH(NHR⁸)-C(O)-]ₙ-X-R⁷-CH(NHR⁸)-C(O)-Y-R⁹-OH (V)

wherein X is oxygen or sulfur, R⁷ and R⁹ are independently selected from an alkyl moiety having from 1 to about 6 carbon atoms, aryl, or alkaryl having from 7 to about 12 carbon atoms; R⁸ is hydrogen or an alkyl moiety having from 1 to about 6 carbon atoms, Y is oxygen or -NH-, and n is an integer of from 1 to about 10.

A compound of formula V can be prepared, for example, by forming an oligomer of an amino acid such as, but not limited to, tyrosine (α-amino-β-(4-hydroxyphenyl)propionic acid), serine (α-amino-β-hydroxypropionic acid), threonine (α-amino-β-hydroxybutyric acid), or cysteine (α-amino-β-mercaptopropionic acid), and reacting the oligomer with a diol having the formula HO-R⁹-OH. Preferably, derivatives of tyrosine, serine, threonine, or cysteine are used in which R⁸ is an alkyl moiety having from 1 to about 6 carbon atoms. Preferably, at least one of R⁷ and R⁹ is an aryl moiety such as phenyl. Alternatively, the amino acid can be reacted with an amino alcohol such as ethanolamine.

The compound of formula I or V is mixed with a polyalkylene glycol and/or an absorbable elastomeric polymer derived from the reaction between a dialkylene glycol and one or more cyclic bioabsorbable monomers selected from trimethylene carbonate, dioxanone, and caprolactone. Preferably, the polyalkylene glycol is selected from polyethylene glycol ("PEG"), polypropylene glycol ("PPG"), and poly(ethylene-propylene) glycol ("poly(EG-PG)"). The bioabsorbable elastomeric polymer is preferably formed by the reaction of diethylene glycol (HOCH₂CH₂OCH₂CH₂OH) with trimethylene carbonate. Optionally, the cyclic bioabsorbable monomer can further include up to about 65% by weight of lactide and/or glycolide.

The mixture is then reacted with a biocompatible and bioabsorbable diisocyanate to form a bioabsorbable polymer with urethane linkages. It is an important feature that the in vivo degradation products of the materials and final product are biocompatible. A suitable diisocyanate for use in the polymer described herein is lysine ethyl ester diisocyanate having the formula 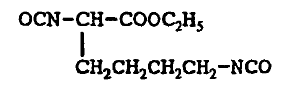 Other amino acid derivatives may alternatively be used.

The bioabsorbable materials disclosed herein may used to fabricate various surgical devices by any of the processes conventionally used to form polymeric materials such as extrusion, drawing, molding, and the like. For example, the bioabsorbable materials may be employed to form filaments for monofilament or multifilament sutures. The filaments may be woven to form sheets which may be used to form various prosthetic devices such as vascular grafts, muscle grafts, bone filler, etc. Alternatively, the filaments can be formed into non-woven fabrics by lamination with or without additional polymeric matrix. Such non-woven fabrics may be used for the same purposes as the woven fabrics listed above.

The bioabsorbable copolymer of the present invention may also be used to form prosthetic devices, surgical clips, surgical fasteners, staples, bone pins, bone screws, anastomosis rings, wound dressings, drug delivery devices, etc.

Optionally, the bioabsorbable material disclosed herein as well as surgical devices made therefrom, can be coated or impregnated with one or more materials which enhance its functionality, e.g., surgically useful substances, such as those which accelerate or beneficially modify the healing process when the material is implanted within a living organism. Thus, for example, antimicrobial agents such as broad spectrum antibiotics (gentamicin sulphate, erythromycin or derivatized glycopeptides) which are slowly released into the tissue can be incorporated to aid in combating clinical and sub-clinical infections in a surgical or trauma wound site.

To promote wound repair and/or tissue growth one or several substances can be introduced into the absorbable material disclosed herein or impregnated into fabrics or prostheses made from bioabsorbable material. Exemplary substances include polypeptides such as human growth factors. The term "human growth factor" or "HGF" embraces those materials, known in the literature, which are referred to as such and includes their biologically active, closely related derivatives. The HGFs can be derived from naturally occurring sources and are preferably produced by recombinant DNA techniques. Specifically, any of the HGFs which are mitogenically active and, as such, effective in stimulating, accelerating, potentiating or otherwise enhancing the wound healing process are useful herein. Growth factors contemplated for use in the materials of the present invention include hEGF (urogastrone), TGF-beta, IGF, PDGF, FGF, etc. These growth factors, methods by which they can be obtained and methods and compositions featuring their use to enhance wound healing are variously disclosed, inter alia, in U.S. Patent Nos. 3,883,497; 3,917,824; 3,948,875; 4,338,397; 4,418,691; 4,528,186, 4,621,052; 4,743,679 and 4,717,717; European Patent Applications 0 046 039; 0 128 733; 0 131 868; 0 136 490; 0 147 178; 0 150 572; 0 177 915 and 0 267 015; PCT International Applications WO 83/04030; WO 85/00369; WO 85/01284 and WO 86/02271 and UK Patent Applications GB 2 092 155 A; 2,162,851 A and GB 2 172 890 A, all of which are incorporated by reference herein. When incorporating wound healing substances such as those discussed above, it may be advantageous to use composite materials having at least one shell layer are formed from a bioabsorbable material having a relatively high rate of bioabsorption. By incorporating wound healing substances in a high rate bioabsorption layer, the substance will be more quickly absorbed while the remaining composite material will still retain sufficiently good mechanical properties to perform its surgical function.

To decrease abrasion, increase lubricity, etc., the bioabsorbably composite materials can be coated with various substances. Copolymers of glycolide and lactide and polyethylene oxide, calcium salts such as calcium stearate, compounds of the pluronic class, copolymers of caprolactone, caprolactone with polyethylene oxide (PEO), polyhydroxyethylmethacrylate (polyHEMA), etc. may be employed.

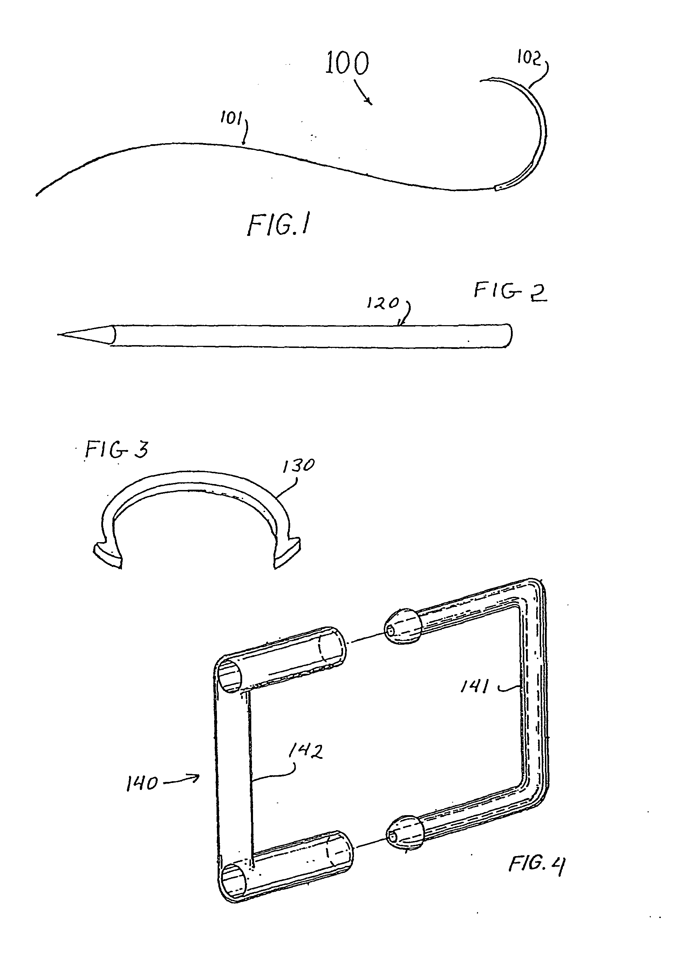

Referring now to the drawings, FIG. 1 illustrates a needle suture combination 100 which includes a needle 102 and a suture 101 fabricated from the bioabsorbable block copolymer described herein.

FIG. 2 illustrates a surgical bone pin 120 fabricated from the bioabsorbable block copolymer described herein.

FIG. 3 illustrates a surgical clip 130 fabricated from the bioabsorbable block copolymer described herein.

FIG. 4 illustrates a two-part surgical fastener 140 fabricated from the bioabsorbable block copolymer described herein. The surgical fastener 140 includes a staple portion 141 with a corresponding retainer 142 with which the staple portion 141 can be engaed

It will be understood that various modifications may be made to the embodiments disclosed herein. Therefore, the above description should not be construed as limiting, but rather as exemplifications of preferred embodiments. Those skilled in the art will envision other modifications within the scope and spirit of the claims appended hereto.

## Claims

1. A bioabsorbable block copolymer possessing hard and soft segments comprising the reaction product of:
a) a biodegradable and biocompatible diisocyanate; and
b) a mixture of
i) an aromatic compound having molecular formula (I): 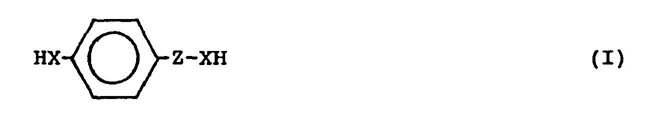 wherein X is oxygen or sulfur, and Z is selected from the group consisting of all alkyl moiety having from 1 to about 6 carbon atoms, an aralkyl moiety having from 7 to about 12 carbon atoms, a moiety having molecular formula (II): 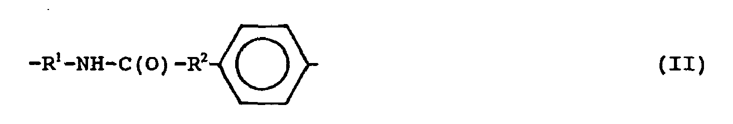 a moiety having molecular formula (III) 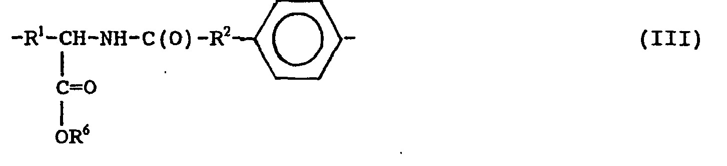 and a moiety having molecular formula (IV):
-R³-CH(NHR⁵)-C(O)O-R⁴- (IV)
wherein R¹, R², R³, and R⁴ are independently selected from the group consisting of an alkyl moiety having from 1 to about 6 carbon atoms and an aralkyl group having from 7 to about 10 carbon atoms, R⁵ is hydrogen or an alkyl group having from 1 to about 6 carbon atoms, and R⁶ is an alkyl group of from about 1 to about 18 carbon atoms; and
ii) an aliphatic polyether glycol and/or an absorbable elastomeric polymer derived from the reaction between dialkylene glycol and a cyclic bioabsorbable monomer including at least one of trimethylene carbonate, dioxanone or caprolactone.

2. The bioabsorbable block copolymer of claim 1 wherein the biodegradable and biocompatible diisocyanate is a lysine derivative.

3. The bioabsorbable block copolymer of claim 2 wherein the lysine derivative is lysine ethyl ester diisocyanate.

4. The bioabsorbable block copolymer of claim 1 wherein R¹ and R² are each -CH₂CH₂-.

5. The bioabsorbable block copolymer of claim 1 wherein the polyalkylene glycol is selected from the group consisting of polyethylene glycol and polypropylene glycol.

6. The bioabsorbable block copolymer of claim 1 wherein the absorbable elastomeric polymer is derived from the reaction between diethylene glycol and trimethylene carbonate.

7. The bioabsorbable block copolymer of claim 1 further containing a medically useful substance selected from the group consisting of antimicrobial agents and tissue growth promoters.

8. A surgical device fabricated from the block copolymer of claim 1.

9. The surgical device of claim 8 wherein the surgical device is selected from the group consisting of a surgical suture, a bone pin, a surgical clip and a two-part surgical fastener.

10. A bioabsorbable block copolymer possessing alternating hard and soft segments comprising the reaction product of:
a) a biodegradable and biocompatible diisocyanate; and
b) a mixture of
i) an amino acid derivative having molecular formula (V):
H-[-X-R⁷-CH(NHR⁸)-C(O)-]ₙ-X-R⁷-CH(NHR⁸)-C(O)-Y-R⁹-OH (V)
wherein X is oxygen or sulfur, R⁷ and R⁹ are independently selected from an alkyl moiety having from 1 to about 6 carbon atoms, aryl, or alkaryl having from 7 to about 12 carbon atoms; R⁸ is hydrogen or an alkyl moiety having from 1 to about 6 carbon atoms; Y is oxygen or -NH-, and n is an integer of from 1 to about 10; and
ii) an aliphatic polyether glycol and/or an absorbable elastomeric polymer derived from the reaction between dialkylene glycol and a cyclic bioabsorbable monomer including at least one of trimethylene carbonate, dioxanone or caprolactone.

11. The bioabsorbable block copolymer of claim 10 wherein the biodegradable and biocompatible diisocyanate is a lysine derivative; preferably the lysine derivative is lysine ethyl ester diisocyanate.

12. The bioabsorbable block copolymer of claim 10 wherein the polyalkylene glycol is selected from the group consisting of polyethylene glycol and polypropylene glycol; or wherein the absorbable elastomeric polymer is derived from the reaction between diethylene glycol and trimethylene carbonate.

13. The bioabsorbable block copolymer of claim 10 further containing a medically useful substance selected from the group consisting of antimicrobial agents and tissue growth promoters.

14. A surgical device fabricated from the block copolymer of claim 10.

15. The surgical device of claim 14 wherein the surgical device is selected from the group consisting of a surgical suture, a bone pin, a surgical clip and a two-part surgical fastener.
